# EUROPÄISCHE PATENTSCHRIFT

(11) **EP 0 741 121 B1**
(45) Veröffentlichungstag und Bekanntmachung des Hinweises auf die Patenterteilung: **14.07.1999**
(21) Anmeldenummer: 96106407.8
(22) Anmeldetag: 24.04.1996
(51) Int. Cl.: C07C 45/89, C07C 49/88, C07D 305/12

(54) **Verfahren zur Herstellung von lösungsmittelarmen Alkyldiketenen**
Process for the preparation of alkyldiketenes containing very few solvents
Procédé pour la préparation d'alkyldicétènes contenant très peu de solvant

(30) Priorität: 03.05.1995 DE 19516183
(43) Veröffentlichungstag der Anmeldung: 06.11.1996
(73) Patentinhaber: BASF Aktiengesellschaft, 67063 Ludwigshafen (DE)
(72) Erfinder: Ettl, Roland, Dr., 67454 Hassloch (DE); Kasel, Wolfgang, Dr., 69226 Nussloch (DE); Fankhänel, Matthias, Dr., 67061 Ludwigshafen (DE); Reuther, Wolfgang, Dr., 69118 Heidelberg (DE)

(56) Entgegenhaltungen:
- EP-A- 0 619 289
- DE-A- 2 927 118

## Beschreibung

Die vorliegende Erfindung betrifft ein Verfahren zur Herstellung von lösungsmittelarmen Alkyldiketenen, die durch Umsetzung von Carbonsäurechloriden mit tertiären Aminen in einem mit Wasser im wesentlichen nicht mischbaren, inerten Lösungsmittel entstehen, durch Destillation zuerst ohne, dann mit Wasser oder Wasserdampf.

Alkyldiketene (AKD) werden überwiegend als Hydrophobierungsmittel in der Papierherstellung verwendet. Üblicherweise überführt man dazu Alkyldiketene, eine wachsartige, in Wasser unlösliche Substanz in eine Dispersion unter Verwendung von ionisch modifizierter Stärke oder synthetischen Polymeren (anionischen oder kationischen) als Schutzkolloid.

Aus der DE-A-23 27 988 ist die Herstellung von Alkyldiketenen durch Umsetzung von Fettsäurechloriden mit tertiären Aminen, bevorzugt Triethylamin, in wasserfreien Lösungsmitteln, z.B. Toluol, und anschließende Extraktion des Amin-Hydrochlorids mit einer Neutralsalzlösung geringer Konzentration bekannt.

Aus der DE-A-29 27 118 ist die Herstellung von Alkyldiketenen durch Umsetzung von Fettsäurechloriden mit tertiären Aminen, bevorzugt eine Mischung aus Dimethylcyclohexylamin und Trimethylamin, in wasserfreien Lösungsmitteln, z.B. Toluol, bekannt. Neben der umständlichen und aufwendigen Handhabung von zwei unterschiedlichen tertiären Aminen gestaltet sich die Aufarbeitung und guantitative Rückführung der beiden Amine schwierig.

In beiden Fällen enthalten die erhaltenen Alkyldiketene nach der destillativen Entfernung noch beachtliche Restmengen an Lösungsmittel (ca. 1%). Eine Verringerung läßt sich nur unter großen technischen Anstrengungen (Hochvakuum, hohe Temperaturen) erzielen. Demgegenüber steht jedoch die mangelnde Thermostabilität (Zersetzung zu polymeren Produkten).

Aus der EP-A-550 107 ist ein Verfahren bekannt, in dem Carbonsäurechloride in Abwesenheit von Lösungsmitteln mit tertiären Aminen umgesetzt werden. Die hierfür beschriebenen Reaktionsapparaturen sind technisch sehr aufwendig und nur für diesen Prozeß zu verwenden. Darüberhinaus gestaltet sich die Aufarbeitung zeitaufwendiger. Bei der wäßrigen Extraktion des Amin-Hydrochlorids ist die Phasentrennung aufgrund der höhen Viskosität der organischen Phase (= Alkyldiketene) langwierig. Bei einer unvollständigen Umsetzung zu Alkyldiketenen entstehen zudem sehr stabile Emulsionen, die zu einer niedrigen Ausbeute führen und damit das Verfahren unwirtschaftlicher machen.

Aus der WO-A-94/18389 ist ein Verfahren bekannt, in dem durch Zusatz von Adsorbentien Reste an organischen Lösungsmitteln entfernt werden. Dazu werden zunächst wäßrige Dispersionen von Alkyldiketenen hergestellt und anschließend mit geeigneten Adsorbentien, z.B. Zeolithen, versetzt, welche das organische Lösungsmittel aufnehmen. Dieses Verfahren ist umständlich, da die zugesetzten Adsorbentien nach der Aufnahme des organischen Lösungsmittels wieder durch Filtration entfernt werden müssen.

Der vorliegenden Erfindung lag daher die Aufgabe zugrunde, den zuvor genannten Nachteilen abzuhelfen.

Demgemäß wurde ein neues und verbessertes Verfahren zur Herstellung von lösungsmittelarmen Alkyldiketenen der allgemeinen Formel I 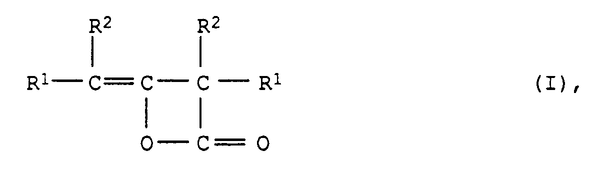 in der
- R¹: C₁₀- bis C₃₀-Alkyl oder C₁₀- bis C₃₀-Alkenyl und
- R²: Wasserstoff oder C₁- bis C₈-Alkyl
bedeuten, durch Umsetzung von Carbonsäurechloriden der allgemeinen Formel II 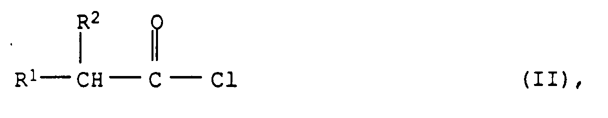 in der R¹ und R² die obengenannten Bedeutungen haben, mit tertiären Aminen der allgemeinen Formel III 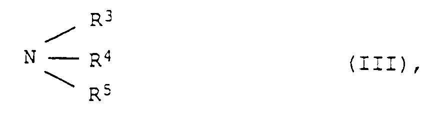 in der
- R³,R⁴,R⁵: C₁- bis C₁₂-Alkyl oder C₃- bis C₁₂- Cycloalkyl,
- R³ und R⁴: gemeinsam eine gegebenenfalls durch Sauerstoff oder Stickstoff unterbrochene C₂- bis C₁₁-Alkylendikette,
bedeuten, in einem mit Wasser im wesentlichen nicht mischbaren, inerten Lösungsmittel gefunden, welches dadurch gekennzeichnet ist, daß man bei Temperaturen von 80 bis 130°C und Drücken von 10 bis 500 mbar und anschließend unter Zusatz von Wasser oder Wasserdampf destilliert.

Das erfindungsgemäße Verfahren kann wie folgt durchgeführt werden:

Man kann ein Carbonsäurechlorid II mit einem tertiärem Amin III in einem mit Wasser im wesentlichen nicht mischbaren inerten organischem Lösungsmittel, bevorzugt im wesentlichen wasserfreien inerten organischem Lösungsmittel, besonders bevorzugt in einem wasserfreien inerten organischem Lösungsmittel bei Temperaturen von 40 bis 95°C, bevorzugt 50 bis 80°C, besonders bevorzugt 60 bis 65°C und Drücken von 0,1 bis 3 bar, bevorzugt 0,5 bis 1,5 bar, besonders bevorzugt Normaldruck (Atmosphärendruck) umsetzen und anschließend nach wäßriger Extraktion die organische Phase destillativ unter einem Druck von 10 bis 500 mbar, bevorzugt 30 bis 350 mbar, besonders bevorzugt 50 bis 250 mbar und Temperaturen von 80 bis 130°C, bevorzugt 95 bis 120°C, besonders bevorzugt 100 bis 115°C das Lösungsmittel entfernen. Daran anschließend kann man unter einem Druck von 10 bis 500 mbar, bevorzugt 30 bis 350 mbar, besonders bevorzugt 50 bis 250 mbar und Temperaturen von 80 bis 130°C, bevorzugt 95 bis 120°C, besonders bevorzugt 100 bis 115°C Wasserdampf oder Wasser in den Reaktionskessel dosieren.

Die Dosierung von Wasserdampf erfolgt in der Regel bevorzugt direkt in die Alkyldiketen-Lösungsmittel-Lösung. Die Dosierung von Wasser erfolgt entweder von unten oder oben in die Alkyldiketen-Lösungsmittel-Lösung oder von oben auf die Oberfläche der Alkyldiketen-Lösungsmittel-Lösung. Die Dosierung kann einfach über ein Einleitungsrohr, bevorzugt nahe dem Rührer erfolgen oder auch über aufwendigere Konstruktionen (perforierte Ringleitung, etc.).

Das Azeotrop aus Wasser und organischem Lösungsmittel wird in der Regel kondensiert und aufgetrennt in eine wäßrige Phase (evtl. mit Spuren an Lösungsmittel verunreinigt) und eine organische Phase. Beide Phasen lassen sich beim nächsten Reaktionsansatz wieder einsetzen.

Nach der Dosierung der gewünschten Wasser- bzw. Dampfmenge wird in der Regel die Destillation noch etwas fortgesetzt (ca. 10 bis 45 min unter Vakuum von 10 bis 50 mbar und 100 bis 120°C). Dann wird das Reaktionsgefäß in der Regel mit Stickstoff belüftet und abgekühlt.

Der Lösungsmittelgehalt im Alkyldiketen kann mit den üblichen analytischen Methoden (z.B. GC) bestimmt werden.

Als mit Wasser im wesentlichen nicht mischbaren, inerte Lösungsmittel eignen sich Alkane, beispielsweise C₅- bis C₃₀-Alkane wie n-Pentan, iso-Pentan, Pentangemische, n-Hexan, iso-Hexan, Hexangemische, n-Heptan, n-Octan, bevorzugt n-Hexan, i-Hexan, Hexangemische, n-Heptan-Gemische, n-Octan-Gemische, besonders bevorzugt Hexangemische, Cycloalkane, beispielsweise C₅- bis C₁₂-Cycloalkane wie Cyclopentan, Cyclohexan, Cycloheptan, Cyclooctan, Methylcyclohexan, Ethylcyclohexan, bevorzugt Cyclohexan und methylcyclohexan, besonders bevorzugt Cyclohexan, aromatische Kohlenwasserstoffe wie Benzol, Toluol, die Xylole, bevorzugt Benzol und Toluol, besonders bevorzugt Toluol, chlorierte Alkane z.B. 1,2-Dichlorpropan, 1,2-Dichlorbutan, bevorzugt 1,2-Dichlorpropan, Ether, beispielsweise Diethylether, Di-isopropylether, Di-isoamylether, Di-n-hexylether, Methyl-t-butylether, bevorzugt Diisopropylether und Methyl-t-butylether oder deren Gemische.

Das Molverhältnis von Amin III zum Carbonsäurechlorid II beträgt in der Regel 0,1:1 bis 30:1, bevorzugt 1:1 bis 3:1, besonders bevorzugt 1:1 bis 1,2:1.

Das Gewichtsverhältnis von inertem Lösungsmittel zum Carbonsäurechlorid II beträgt in der Regel 0,01:1 bis 100:1, bevorzugt 0,5:1 bis 2:1, besonders bevorzugt 0,8:1 bis 1:1.

Benötigte Wassermenge bzw. Dampfmenge beträgt in der Regel 3 bis 300 Gew.-%, bevorzugt 5 bis 50 Gew.-%, besonders bevorzugt 5 bis 30 Gew.-% bezogen auf das Alkyldiketen I.

Die Carbonsäurechloride I sind in der Regel längerkettige gesättigte oder ungesättigte Säurechloride mit 12 und mehr C-Atomen, bevorzugt mit 16 und mehr C-Atomen; auch Gemische von Säurechloriden aus natürlich gewonnenen Fettsäuren können eingesetzt werden wie z.B. die Fettsäuren aus Kokosöl, Tallöl, Ricinusöl, Olivenöl, Rindertalg, Palmkernfett, bevorzugt sind Fettsäure-Gemische mit C₁₆- bis C₂₂, besonders bevorzugt C₁₆- bis C₁₈.

Die Substituenten R¹, R², R³, R⁴ und R⁵ in den Verbindungen I, II und III haben folgende Bedeutungen:
R¹
   - C₁₀- bis C₃₀-Alkyl, bevorzugt C₁₂- bis C₂₅-Alkyl, besonders bevorzugt C₁₂- bis C₂₀-Alkyl wie Dodecyl, Tetradecyl und Hexadecyl,
   - C₁₀- bis C₃₀-Alkenyl, bevorzugt C₁₂- bis C₂₅-Alkenyl, besonders bevorzugt C₁₂- bis C₂₀-Alkenyl wie Tetradec-7-1-yl und Hexadec-7-1-yl,
R²
   - Wasserstoff,
   - C₁- bis C₈-Alkyl, bevorzugt C₁- bis C₄-Alkyl wie Methyl, Ethyl, n-Propyl, iso-Propyl, n-Butyl, iso-Butyl, sec.-Butyl, tert.-Butyl, besonders bevorzugt Methyl und Ethyl,
R³, R⁴, R⁵
   - C₁- bis C₁₂-Alkyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, besonders bevorzugt C₁- bis C₄-Alkyl wie Methyl, Ethyl, n-Propyl, iso-Propyl, n-Butyl, iso-Butyl, sec.-Butyl und tert.-Butyl,
   - C₃- bis C₁₂-Cycloalkyl, bevorzugt C₃- bis C₈-Cycloalkyl wie Cyclopropyl, Cyclobutyl, Cyclopentyl, Cyclohexyl, Cycloheptyl und Cyclooctyl, besonders bevorzugt Cyclopentyl, Cyclohexyl und Cyclooctyl,
R³, R⁴
   - gemeinsam eine gegebenenfalls durch Sauerstoff oder Stickstoff unterbrochene C₂- bis C₁₁-Alkylendikette, bevorzugt eine gegebenenfalls durch Sauerstoff oder Stickstoff unterbrochene C₃- bis C₇-Alkylendikette, wie -(CH₂)₃-, -(CH₂)₄-, -(CH)₂-, -(CH₂)₅-, -(CH₂)₂-N-(CH₂)₂- und -(CH₂)₂-O-(CH₂)₂-.

Verwendung des "lösungsmittelfreien" AKD's: zur Hydrophobierung von Papier; das AKD mit geringem Lösungsmittelgehalt hat Vorteile bei der Lagerstabilität (Toluol beeinflußt die Lagerstabilität negativ), bei der Anwendung in der Papierfabrik (in der Trockenpartie der Papierherstellung entweicht das Lösungsmittel) und in der späteren Verwendung der so hergestellten Papiere (z.B. bei der Getränkekartonherstellung (Tetrapak) werden wegen dem Kontakt mit Lebensmittel keine Lösungsmittel toleriert).

Das erfindungsgemäße Verfahren läßt sich auch auf andere organische Substanzen ausdehnen, die aufgrund ihrer Struktur prinzipiell mit Wasser reagieren können, gelöst in einem organischen Lösungsmittel anfallen, destillativ gereinigt werden sollen und die Notwendigkeit eines niedrigen Lösungsmittelgehalts bestehen: z.B. Anhydride, lmide von langkettigen Fettsäuren, gemischte Anhydride oder Imide von langkettigen Fettsäuren mit anderen Carbonsäuren/Carbonsäurederivate z.B. acylierte Caprolactame und acylierte Benzoesäuren.

### Beispiele

### Beispiel 1

Zu 230 g Dimethylcyclohexylamin in 400 g Toluol wurden 480 g Stearinsäurechlorid bei einer Temperatur von 60°C zudosiert und 1h nachgerührt. Anschließend wurde mit Schwefelsäure angesäuert und 2 mal mit Wasser extrahiert. Der überwiegende Teil des Lösungsmittels der organischen Phase wurde bei Temperaturen von 100 bis 110°C i. Vak. (100 mbar) abdestilliert und anschließend unter Beibehaltung der Temperatur und des Vakuums insgesamt 240 g Wasserdampf eingeleitet. Die Destillation wurde noch für 30 min fortgesetzt, bevor mit Stickstoff belüftet und abgekühlt wurde. Die Ausbeute betrug 97,3% mit einem Alkyldiketen-Gehalt von 92,6%.

### Beispiel 2

Zu 160 g Triethylamin in 520 g Toluol wurden 445 g Stearinsäurechlorid bei einer Temperatur von 65°C zudosiert und 1h nachgerührt. Anschließend wurde mit Schwefelsäure angesäuert und 2 mal mit Wasser extrahiert. Der überwiegende Teil des Lösungsmittels der organischen Phase wurde bei Temperaturen von 100 bis 110°C i. Vak. (145 mbar) abdestilliert und anschließend unter Beibehaltung der Temperatur und des Vakuums insgesamt 120 g Wasserdampf eingeleitet. Die Destillation wurde noch für 30 min fortgesetzt, bevor mit Stickstoff belüftet und abgekühlt wurde. Die Ausbeute betrug 97,3% mit einem Alkyldiketen-Gehalt von 92,6%.

### Beispiel 3

Zu 214 g Triethylamin in 530 g Toluol wurden 560 g Stearinsäurechlorid bei einer Temperatur von 65°C zudosiert und 1h nachgerührt. Anschließend wurde mit Schwefelsäure angesäuert und 2 mal mit Wasser extrahiert. Der überwiegende Teil des Lösungsmittels der organischen Phase wurde bei Temperaturen von 100 bis 110°C i. Vak. (70 mbar) abdestilliert und anschließend unter Beibehaltung der Temperatur und des Vakuums insgesamt 180 g Wasser zudosiert. Die Destillation wurde noch für 30 min fortgesetzt, bevor mit Stickstoff belüftet und abgekühlt wurde. Die Ausbeute betrug 99,1% mit einem Alkyldiketen-Gehalt von 91,5%.

Toluolgehalt der hergestellten Alkyldiketene:
(Bestimmung über GC)

| Beispiel-Nr. | Toluolgehalt [ppm] |
|---|---|
| 1 | < 2 |
| 2 | 5 |
| 3 | 8 |
| Vergleich A | 7350 |
| Vergleichsbeispiel A: nach DE-A-29 27 118, Beispiel Nr.: 4 | |

Zu 245 g Dimethylcyclohexylamin in 500 g Toluol wurden 500 g Stearinsäurechlorid bei einer Temperatur von 60°C zudosiert und 1h nachgerührt. Anschließend wurde mit Schwefelsäure angesäuert und 2 mal mit Wasser extrahiert und die organische Phase destillativ (Destillation: 100 bis 110°C, 15 mbar) von Toluol befreit. Die Ausbeute betrug 97% mit einem Alkyldiketen-Gehalt von 86,7%.

## Patentansprüche

1. Verfahren zur Herstellung von lösungsmittelarmen Alkyldiketenen der allgemeinen Formel I 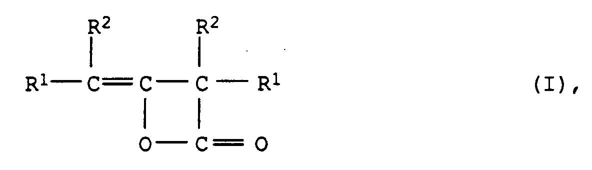 in der
R¹ C₁₀- bis C₃₀-Alkyl oder C₁₀- bis C₃₀-Alkenyl und
R² Wasserstoff oder C₁- bis C₈-Alkyl
bedeuten, durch Umsetzung von Carbonsäurechloriden der allgemeinen Formel II 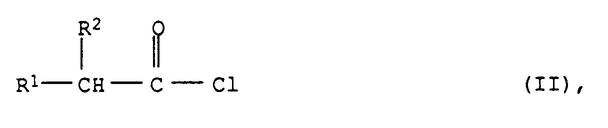 in der R¹ und R² die obengenannten Bedeutungen haben, mit tertiären Aminen der allgemeinen Formel III 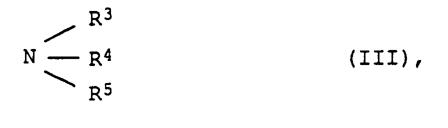 in der
R³,R⁴,R⁵ C₁- bis C₁₂-Alkyl oder C₃- bis C₁₂-Cycloalkyl,
R³ und R⁴ gemeinsam eine gegebenenfalls durch Sauerstoff oder Stickstoff unterbrochene C₂- bis C₁₁-Alkylendikette,
bedeuten, in einem mit Wasser im wesentlichen nicht mischbaren, inerten Lösungsmittel, dadurch gekennzeichnet, daß man bei Temperaturen von 80 bis 130°C und Drücken von 10 bis 500 mbar und anschließend unter Zusatz von Wasser oder Wasserdampf destilliert.

2. Verfahren zur Herstellung von lösungsmittelarmen Alkyldiketenen I nach Anspruch 1, dadurch gekennzeichnet, daß R¹ C₁₂- bis C₂₅-Alkyl oder C₁₂- bis C₂₅-Alkenyl bedeutet.

3. Verfahren zur Herstellung von lösungsmittelarmen Alkyldiketenen I nach Anspruch 1, dadurch gekennzeichnet, daß R¹ C₁₄- bis C₂₀-Alkyl oder C₁₄- bis C₂₀-Alkenyl bedeutet.

4. Verfahren zur Herstellung von lösungsmittelarmen Alkyldiketenen I nach den Ansprüchen 1 bis 3, dadurch gekennzeichnet, daß R² Wasserstoff bedeutet.

5. Verfahren zur Herstellung von lösungsmittelarmen Alkyldiketenen I nach Anspruch 1, dadurch gekennzeichnet, daß man die Destillationen bei Temperaturen von 95 bis 120°C und Drücken von 30 bis 350 mbar durchführt.

6. Verfahren zur Herstellung von lösungsmittelarmen Alkyldiketenen I nach Anspruch 1, dadurch gekennzeichnet, daß man die Destillationen bei Temperaturen von 100 bis 115°C und Drücken von 50 bis 250 mbar durchführt.

## Claims

1. A process for the preparation of low-solvent alkyl ketene dimers of the formula I 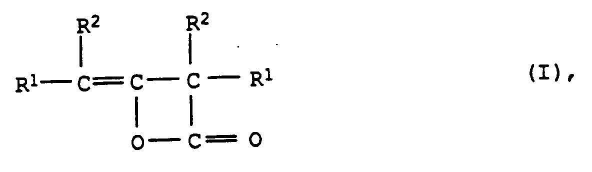 where
R¹ is C₁₀- to C₃₀-alkyl or C₁₀- to C₃₀-alkenyl, and
R² is hydrogen or C₁- to C₈-alkyl,
by reacting acyl chlorides of the formula II 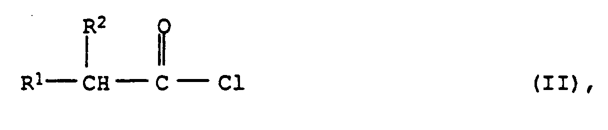 where R¹ and R² are as defined above, with tertiary amines of the formula III 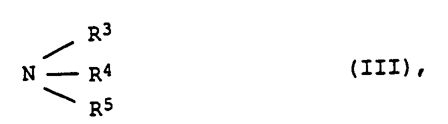 where
R³,R⁴ and R⁵ are C₁- to C₁₂-alkyl or C₃- to C₁₂-cycloalkyl, and
R³ and R⁴ together are a C₂- to C₁₁-alkylene dimer chain, which may be interrupted by oxygen or nitrogen,
in an essentially water-immiscible, inert solvent, which comprises distilling the product at from 80 to 130°C and at from 10 to 500 mbar and subsequently with addition of water or steam.

2. A process for the preparation of low-solvent alkyl ketene dimers I as claimed in claim 1, wherein R¹ is C₁₂- to C₂₅-alkyl or C₁₂- to C₂₅-alkenyl.

3. A process for the preparation of low-solvent alkyl ketene dimers I as claimed in claim 1, wherein R¹ is C₁₄- to C₂₀-alkyl or C₁₄- to C₂₀-alkenyl.

4. A process for the preparation of low-solvent alkyl ketene dimers I as claimed in claims 1 to 3, wherein R² is hydrogen.

5. A process for the preparation of low-solvent alkyl ketene dimers I as claimed in claim 1, wherein the distillation is carried out at from 95 to 120°C and at from 30 to 350 mbar.

6. A process for the preparation of low-solvent alkyl ketene dimers I as claimed in claim 1, wherein the distillation is carried out at from 100 to 115°C and at from 50 to 250 mbar.

## Revendications

1. Procédé de préparation d'alkyldicétènes pauvres en solvant de formule générale I 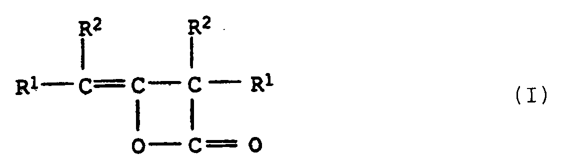 dans laquelle
R¹ représente un groupe alkyle en C₁₀ à C₃₀ ou un groupe alcényle en C₁₀ à C₃₀ et
R² représente un atome d'hydrogène ou un groupe alkyle en C₁ à C₈,
en faisant réagir des chlorures d'acide carbonique de formule générale II 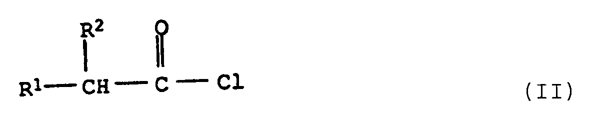 dans laquelle R¹ et R² possèdent les significations précitées, avec des amines tertiaires de formule générale III 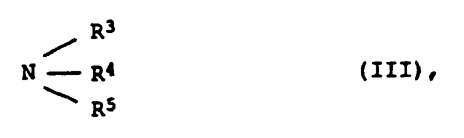 dans laquelle
R³, R⁴, R⁵ représentent des groupes alkyle en C₁ à C₁₂ ou cycloalkyle en C₃ à C₁₂,
R³ et R⁴ représentent ensemble une double chaîne alkylène en C₂ à C₁₁ éventuellement interrompue par un atome d'oxygène ou d'azote,
dans un solvant inerte essentiellement non miscible à l'eau, caractérisé en ce qu'on distille ensuite en ajoutant de l'eau ou de la vapeur d'eau à des températures de 80 à 130°C et à des pressions de 10 à 500 mbar.

2. Procédé de préparation d'alkyldicétènes I pauvres en solvant selon la revendication 1, caractérisé en ce que R¹ représente un groupe alkyle en C₁₂ à C₂₅ ou alcényle en C₁₂ à C₂₅.

3. Procédé de préparation d'alkyldicétènes I pauvres en solvant selon la revendication 1, caractérisé en ce que R¹ représente un groupe alkyle en C₁₄ à C₂₀ ou alcényle en C₁₄ à C₂₀.

4. Procédé de préparation d'alkyldicétènes I pauvres en solvant selon l'une quelconque des revendications 1 à 3, caractérisé en ce que R² représente un atome d'hydrogène.

5. Procédé de préparation d'alkyldicétènes I pauvres en solvant selon la revendication 1, caractérisé en ce que l'on réalise les distillations à des températures de 95 à 120°C et à des pressions de 30 à 350 mbar.

6. Procédé de préparation d'alkyldicétènes I pauvres en solvant selon la revendication 1, caractérisé en ce que l'on réalise les distillations à des températures de 100 à 115°C et à des pressions de 50 à 250 mbar.
